# EUROPÄISCHE PATENTSCHRIFT

(11) **EP 0 772 651 B1**
(45) Veröffentlichungstag und Bekanntmachung des Hinweises auf die Patenterteilung: **30.09.1998**
(21) Anmeldenummer: 95925868.2
(22) Anmeldetag: 13.07.1995
(51) Int. Cl.: C09B 51/00, C07D 271/06

(54) **NITROFARBSTOFFE**
NITRO DYES
COLORANTS NITRES

(30) Priorität: 21.07.1994 DE 4425794
(43) Veröffentlichungstag der Anmeldung: 14.05.1997
(73) Patentinhaber: BASF AKTIENGESELLSCHAFT, 67056 Ludwigshafen (DE)
(72) Erfinder: LAMM, Gunther, D-67454 Hassloch (DE); REICHELT, Helmut, D-67435 Neustadt (DE); GRUND, Clemens, D-68199 Mannheim (DE)
(86) Internationale Anmeldenummer: EP9502745
(87) Internationale Veröffentlichungsnummer: WO9603463

(56) Entgegenhaltungen:
- EP-A- 0 006 171
- EP-A- 0 037 465
- WO-A-89/10384
- FR-A- 2 133 803
- FR-A- 2 451 932
- GB-A- 1 535 401
- US-A- 2 708 149
- CHEMICAL ABSTRACTS, vol. 76, no. 18, 1.Mai 1972 Columbus, Ohio, US; abstract no. 101196t, Seite 84; & JP,A,46 000 432 (DAITO CHEMICAL INDUSTRIAL CO) 7.Januar 1971

## Beschreibung

Die vorliegende Erfindung betrifft neue Nitrofarbstoffe der Formel I 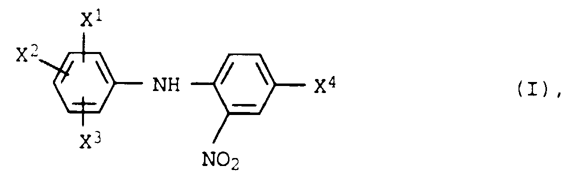 in der
- X¹ und X²: unabhängig voneinander jeweils Wasserstoff, C₁-C₆-Alkyl, C₁-C₆-Alkoxy oder Pyrrolidin-2-on-1-yl,
- X³: Wasserstoff oder C₁-C₆-Alkyl und
- X⁴: einen Rest der Formel 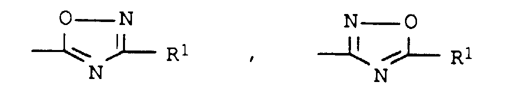 oder 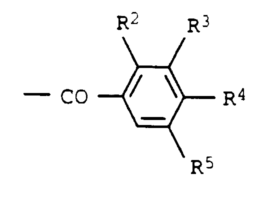 bedeuten, worin
R¹ für Wasserstoff, C₁-C₁₂-Alkyl, das durch 1 bis 3 Sauerstoffatome in Etherfunktion unterbrochen sein kann und gegebenenfalls durch Phenyl, Phenoxy oder Acetyl substituiert ist, C₃-C₇-cycloalkyl, das gegebenenfalls durch C₁-C₄-Alkyl substituiert ist, oder Phenyl, das gegebenenfalls durch C₁-C₄-Alkyl, C₁-C₄-Alkoxy oder Halogen substituiert ist,
R² und R³ unabhängig voneinander jeweils für Wasserstoff, C₁-C₆-Alkyl oder C₁-C₆-Alkoxy,
R⁴ für Wasserstoff, Methyl, Ethyl, Methoxy oder Ethoxy und
R⁵ für Wasserstoff oder C₁-C₆-Alkyl stehen,

Verfahren zu ihrer Herstellung und ihre Verwendung zum Färben oder Bedrucken von textilen Materialien.

Der Nitrofarbstoff C.I. Disperse Yellow 42 (10338) ist handelsüblich. Dabei handelt es sich um 2-Nitro-4-phenylsulfamoyldiphenylamin.

Aufgabe der vorliegenden Erfindung war es nun, neue Nitrofarbstoffe bereitzustellen, die vorteilhafte anwendungstechnische Eigenschaften aufweisen.

Demgemäß wurden die eingangs näher bezeichneten Nitrofarbstoffe der Formel I gefunden.

Alle in der obengenannten Formel auftretenden Alkylgruppen können sowohl geradkettig als auch verzweigt sein.

Wenn in der obengenannten Formel I substituierte Alkyl- oder Cycloalkylgruppen auftreten, so weisen diese in der Regel 1 oder 2 Substituenten auf.

Wenn in der obengenannten Formel I substituierte Phenylgruppen auftreten, so weisen diese in der Regel 1 bis 3, vorzugsweise 1 oder 2 Substituenten auf.

Reste X¹, X², X³, R¹, R², R³ und R⁵ sind z.B. Methyl, Ethyl, Propyl, Isopropyl, Butyl, Isobutyl, sec-Butyl, tert-Butyl, Pentyl, Isopentyl, Neopentyl, tert-Pentyl, Hexyl oder 2-Methylpentyl.

Reste R¹ sind weiterhin z.B. Heptyl, Octyl, 2-Ethylhexyl, Isooctyl, Nonyl, Isononyl, Decyl, Isodecyl, Undecyl oder Dodecyl (die obigen Bezeichnungen Isooctyl, Isononyl und Isodecyl sind Trivialbezeichnungen und stammen von den nach der Oxosynthese erhaltenen Alkoholen - vgl. dazu Ullmann's Encyclopedia of Industrial Chemistry, 5^{th} Edition, Vol. A1, Seiten 290 bis 293, sowie Vol. A10, Seiten 284 und 285), 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- oder 4-Butoxybutyl, 4,8-Dioxadecyl, 4,7-Dioxaundecyl, 3,6,9-Trioxadecyl, 3,6,9-Trioxaundecyl, 3,6,9-Trioxadodecyl, 4,7,10-Trioxaundecyl, Benzyl, 1- oder 2-Phenylethyl, Phenoxymethyl, 1- oder 2-Phenoxyethyl, 2- oder 3-Phenoxypropyl, Acetylmethyl, Cyclopropyl, Cyclobutyl, Cyclopentyl, Methylcyclopentyl, Dimethylcyclopentyl, Cyclohexyl, Methylcyclohexyl, Dimethylcyclohexyl, Cycloheptyl, Phenyl, 2-, 3- oder 4-Methylphenyl, 2,4-Dimethylphenyl, 2-, 3- oder 4-Ethylphenyl, 2,6-Diethylphenyl, 2-, 3- oder 4-Methoxyphenyl, 2,4-Dimethoxyphenyl, 2-, 3- oder 4-Ethoxyphenyl, 2,6-Diethoxyphenyl, 2-, 3- oder 4-Chlorphenyl oder 2,4-Dichlorphenyl.

Reste X¹, X², R² und R³ sind weiterhin z.B. Methoxy, Ethoxy, Propoxy, Isopropoxy, Butoxy, Isobutoxy, sec-Butoxy, tert-Butoxy, Pentyloxy, Isopentyloxy, Neopentyloxy, tert-Pentyloxy oder Hexyloxy.

Bevorzugt sind Nitrofarbstoffe der Formel I, in der X¹ und X² unabhängig voneinander jeweils Wasserstoff oder C₁-C₄-Alkyl und X³ Wasserstoff bedeuten.

Weiterhin bevorzugt sind Nitrofarbstoffe der Formel I, in der X⁴ einen Rest der Formel 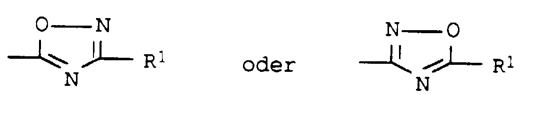 bedeutet, worin R¹ jeweils die obengenannte Bedeutung besitzt.

Weiterhin bevorzugt sind Nitrofarbstoffe, die der Formel Ia 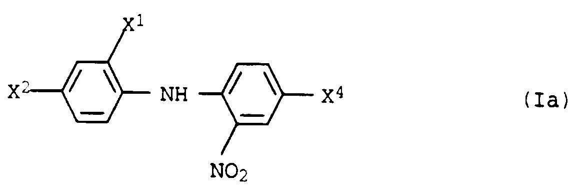 gehorchen, in der X¹, X² und X⁴ jeweils die obengenannte Bedeutung besitzen.

Weiterhin bevorzugt sind Nitrofarbstoffe der Formel I, in der X⁴ einen Rest der Formel 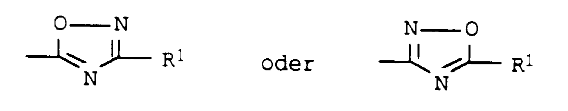 bedeutet, worin R¹ für C₁-C₄-Alkyl, Cyclohexyl oder Phenyl steht.

Besonders bevorzugt sind Nitrofarbstoffe, die der Formel Ia gehorchen, in der X¹ und X² unabhängig voneinander jeweils Wasserstoff oder C₁-C₄-Alkyl und X⁴ einen Rest der Formel 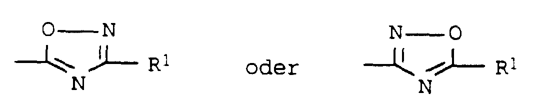 bedeuten, worin R¹ für C₁-C₄-Alkyl, Cyclohexyl oder Phenyl steht.

Besonders bevorzugt sind weiterhin Nitrofarbstoffe der Formel Ia, in der X¹ und X² beide jeweils Wasserstoff oder beide jeweils Methyl oder X¹ Wasserstoff und X² C₁-C₄-Alkyl bedeuten.

Von besonderer Bedeutung sind Nitrofarbstoffe der Formel Ib oder Ic 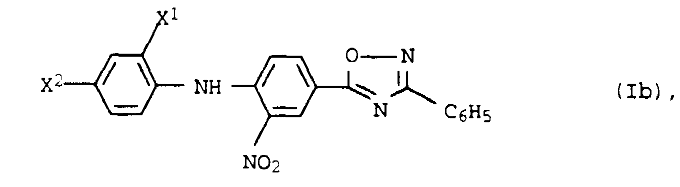 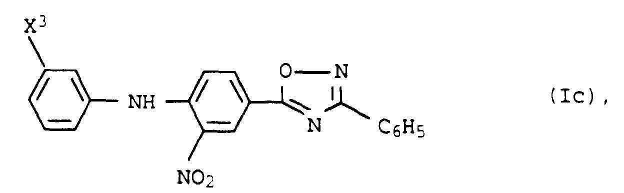 in der
- X¹ und X²: beide jeweils Wasserstoff oder beide jeweils Methyl oder
- X¹: Wasserstoff und X² C₁-C₄-Alkyl und
- X³: C₁-C₄-Alkyl bedeuten.

Die erfindungsgemäßen Nitrofarbstoffe der Formel I können nach an sich bekannten Methoden erhalten werden.

Beispielsweise kann man ein Nitrobenzol der Formel II 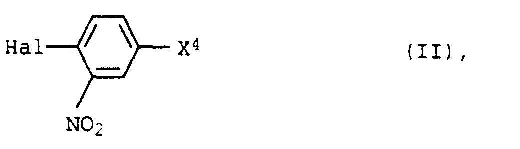 in der Hal Halogen, insbesondere Chlor oder Brom, bedeutet und X⁴ die obengenannte Bedeutung besitzt, mit einem Anilin der Formel III 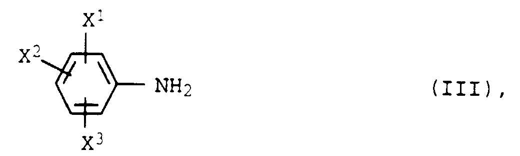 in der X¹, X² und X³ jeweils die obengenannte Bedeutung besitzen, umsetzen.

Diejenigen Nitrofarbstoffe der Formel I, in der X⁴ einen Rest der Formel 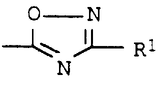 bedeutet, können z.B. erhalten werden, indem man ein Benzoylhalogenid der Formel IV 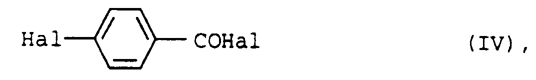 in der Hal die obengenannte Bedeutung besitzt, mit einer Verbindung der Formel V 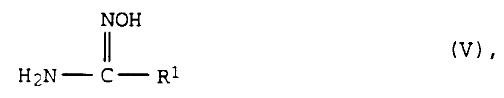 in der R¹ die obengenannte Bedeutung besitzt, zu Verbindungen der Formel VI 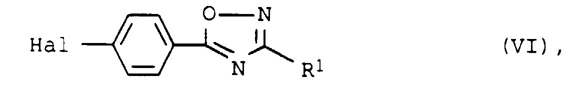 in der R¹ und Hal jeweils die obengenannte Bedeutung besitzen, umsetzt, diese nitriert und anschließend mit dem Anilin der Formel III zur Reaktion bringt.

Diejenigen Nitrofarbstoffe der Formel I, in der X⁴ einen Rest der Formel 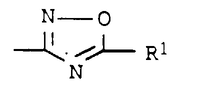 bedeutet, können z.B. erhalten werden, indem man ein Benzamidin der Formel VII 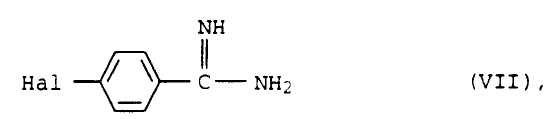 in der Hal die obengenannte Bedeutung besitzt, mit einem Carbonsäurederivat der Formel VIII

R¹―CO―Z (VIII),

in der Z Halogen, insbesondere Chlor, C₁-C₆-Alkoxy oder einen Rest der Formel O―CO―R¹ bedeutet und R¹ jeweils die obengenannte Bedeutung besitzt, umsetzt und die resultierende Verbindung der Formel IX 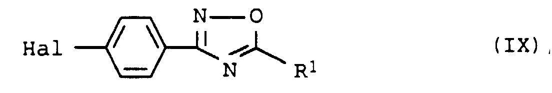 in der Hal und R¹ jeweils die obengenannte Bedeutung besitzen, nitriert und anschließend mit dem Anilin der Formel III zur Reaktion bringt.

Man kann in diesem Fall aber auch von einer Cyanoverbindung der Formel X 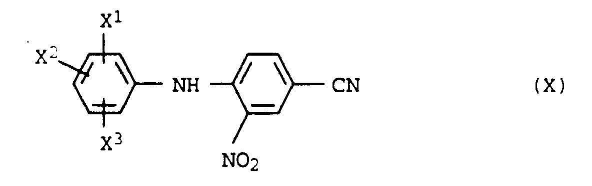 ausgehen, in der X¹, X² und X³ jeweils die obengenannte Bedeutung besitzen, diese mit Hydroxylamin in das Amidoxim der Formel XI 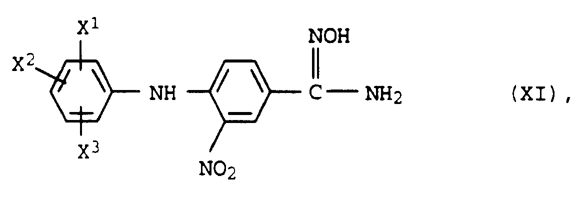 in der X¹, X² und X³ jeweils die obengenannte Bedeutung besitzen, überführen und anschließend mit dem Carbonsäurederivat der Formel VIII zur Reaktion bringen.

Bei der letztgenannten Methode ist es von besonderem Vorteil, wenn man die Bildung des Amidoxims XI sowie die Umsetzung mit dem Carbonsäurederivat VIII in einem Isobutanol/Wasser-Gemisch vornimmt und das Amidoxim XI nicht zwischenisoliert.

Setzt man das Aldoxim der Formel XI mit Diketen um, so erhält man einen Nitrofarbstoff der Formel I, in der X⁴ den Rest der Formel 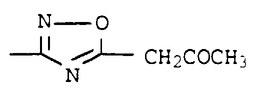 bedeutet.

Die neuen Nitrofarbstoffe der Formel I eignen sich in vorteilhafter Weise zum Färben oder Bedrucken von textilen Materialien.

Geeignete textile Materialien sind beispielsweise Polyester in den verschiedenen Anwendungsformen (z.B. Gewebe, Gewirke, Garn oder Flocke) oder deren Mischungen mit anderen Fasern.

Besonders vorteilhaft ist die Anwendung der Nitrofarbstoffe der Formel I zusammen mit UV-Absorbern. Geeignete UV-Absorber sind z.B. solche der Formel XII oder XIII 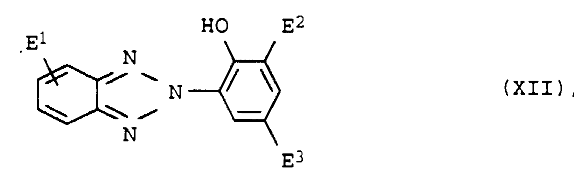 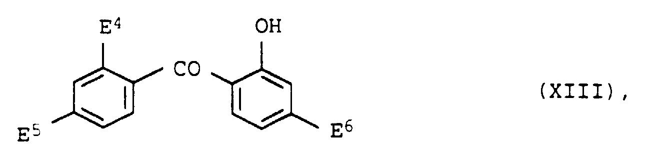 worin
- E¹: Wasserstoff oder Chlor,
- E² und E³: unabhängig voneinander jeweils Wasserstoff, C₁-C₈-Alkyl, das gegebenenfalls durch Phenyl substituiert ist,
- E⁴: Wasserstoff oder Hydroxy und
- E⁵ und E⁶: unabhängig voneinander jeweils Wasserstoff, Hydroxy, C₁-C₆-Alkoxy, das gegebenenfalls durch Hydroxy, C₁-C₄-Alkoxy, C₁-C₄-Alkanoyloxy oder Benzoyloxy substituiert ist, bedeuten.

Besonders zu nennen sind dabei UV-Absorber der Formel XIIa, XIIb, XIIIa oder XIIIb

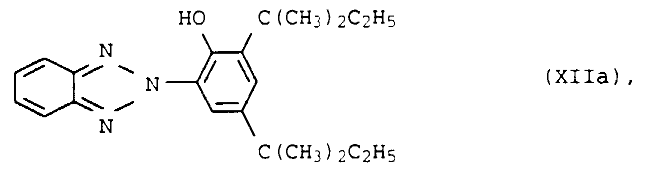

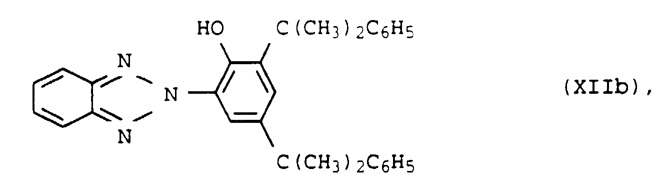

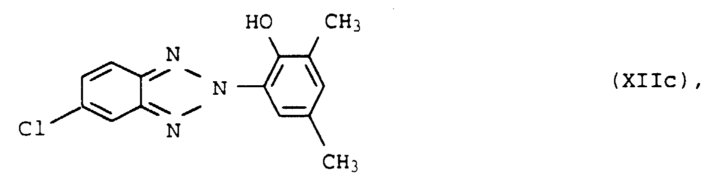

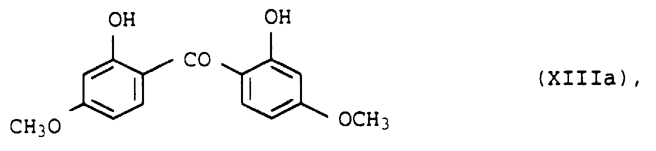

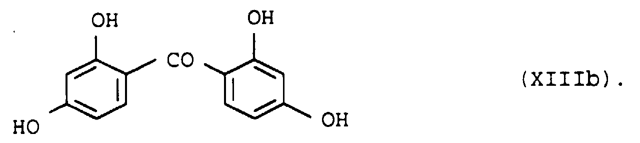

Die UV-Absorber können dabei einzeln oder auch in Mischung untereinander zur Anwendung gelangen. Dabei sind jeweils Mischungen verschiedener Spezies der Formel XII oder XIII oder auch Mischungen von Verbindungen der Formel XII und XIII geeignet.

Im allgemeinen verwendet man dabei 1 bis 8 Gew.-%, vorzugsweise 2 bis 5 Gew.-%, jeweils bezogen auf das Gewicht der Nitrofarbstoffe, an UV-Absorbern.

Die Nitrofarbstoffe der Formel I zeichnen sich durch ein vorteilhaftes anwendungstechnisches Eigenschaftsprofil aus. Insbesondere weisen sie eine sehr gute Heißlichtechtheit auf.

Die folgenden Beispiele sollen die Erfindung näher erläutern.

### Färbevorschrift

Mittels dieser Vorschrift können die im folgenden näher beschriebenen Farbstoffe zur Anwendung gelangen.

10 g Polyestergewebe werden bei einer Temperatur von 50°C in 200 ml einer Färbeflotte gegeben, die 0,5 Gew.-%, bezogen auf das Polyestergewebe, einer Farbstoffpräparation enthält und deren pH-Wert mittels Essigsäure auf 4,5 eingestellt ist. Man behandelt 5 min bei 50°C, steigert dann die Temperatur der Flotte innerhalb von 30 min auf 130°C, hält 60 min bei dieser Temperatur und läßt dann innerhalb von 20 min auf 60°C abkühlen.

Danach wird das ausgefärbte Polyestergewebe reduktiv gereinigt, indem man es 15 min in 200 ml einer Flotte, die 5 ml/1 32 gew.-%ige Natronlauge, 3 g/l Natriumdithionit und 1 g/l eines Anlagerungsproduktes von 48 mol Ethylenoxid an 1 mol Ricinusöl enthält, bei 65°C behandelt. Schließlich wird das Gewebe gespült, mit verdünnter Essigsäure neutralisiert, nochmals gespült und getrocknet.

Die obengenannte Farbstoffpräparation enthält 40 Gew.-% Nitrofarbstoff sowie 60 Gew.-% eines Dispergiermittels auf Basis von Ligninsulfonat, jeweils bezogen auf das Gewicht der Präparation. Bezogen auf das Gewicht an Nitrofarbstoff, kann die Farbstoffpräparation weiterhin noch 1 bis 8 Gew.-% eines oben näher bezeichneten UV-Absorbers enthalten.

### Beispiel 1

a) 277 g p-Chlorbenzonitril wurden in einem Gemisch aus 166 g Soda, 600 ml Wasser und 640 ml Isobutanol sowie 1 g eines Schwermetallkomplexbildners und 170 g Hydroxylaminsulfat 5 h bei 90°C gerührt. Danach ließ man bei 40°C absitzen und verwarf die wäßrige Phase. Anschließend setzte man zur erhaltenen Isobutanol-Phase 225 g Acetanhydrid zu. Man rührte 30 min nach, destillierte Isobutanol ab und fällte mit Wasser. Nach dem Isolieren trocknete man Produkt bei 160°C. Ausbeute: 390 g des Produktes der Formel 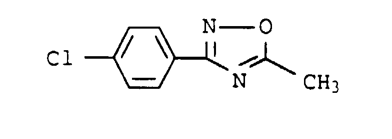
b) 98 g des unter a) beschriebenen Produkts wurden bei maximal 20°C in 250 ml konz. Schwefelsäure gelöst. Dann tropfte man bei ≤ 10°C 25 ml 98 gew.-%ige Salpetersäure hinzu, rührte 10 h nach und fällte auf Eiswasser. Nach Isolieren, Absaugen, 5 Waschen und Trocknen erhielt man 116 g des Produkts der Formel 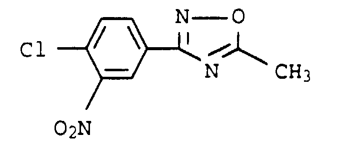 Fp.: 110°C.
c) 25 g des unter b) beschriebenen Produkts wurden mit 70 g m-Toluidin 5 h bei 145 bis 150°C und anschließend noch 2 h bei 160°C gerührt. Anschließend kühlte man das Gemisch auf ca. 100°C ab und gab es unter Rühren zu 1000 ml Wasser und 50 ml konz. Salzsäure. Der ausgefallene gelbe Farbstoff der Formel 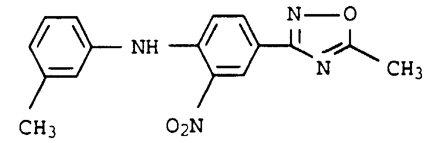 wurde abgesaugt und mit Wasser gewaschen. Nach dem Trocknen erhielt man 31 g eines orangefarbenen Pulvers, das Polyestergewebe gemäß der eingangs beschrieben Färbevorschrift in rotstichig gelbem Ton färbt, wobei die Färbungen hervorragende Echtheiten aufweisen. Insbesondere zeigt der Farbstoff ein gutes Aufziehverhalten auch schon bei 120°C und hat vorzügliche Lichtechtheit. Sein Absorptionsmaximum in einer Lösung von 0,25 g/Liter einer Mischung aus N,N-Dimethylformamid und Eisessig (2:1 v/v) beträgt 432 nm. Der Extinktionskoeffizient des Rohproduktes am Maximum beträgt ca. 24.

### Beispiel 2

196 g Hydroxylaminsulfat wurden in ein Gemisch aus 685 ml Wasser und 132 g Soda gegeben. Dazu gab man einen Komplexbildner zur Entfernung etwa vorhandener Schwermetallspuren und eine Lösung von 235 g Benzonitril in 685 ml Isobutanol. Nach 6-stündigem Rühren bei Siedetemperatur ließ man absitzen und entfernte die (untere) wäßrige Phase. Danacb versetzte man die organische Phase mit 451 g 3-Nitro-4-chlor-benzoylchlorid, gelöst in 1100 ml wasserfreiem Aceton, und ruhrte das Gemisch 45 min bei 30 bis 40°C. Dann wurde mit 5000 ml kaltem Wasser verdünnt und das ausgefallene Produkt abgesaugt (Fp.: 137°C). Man erhitzte es dann auf 190°C, dampfte dabei frei werdendes Wasser ab und erhielt das Produkt der Formel 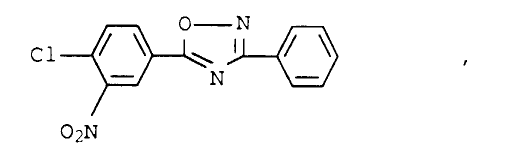 das bei 146°C schmilzt. Man setzte dies analog Beispiel 1 mit Anilin bei 150 bis 160°C um und erhielt dann das orangefarbene Pulver der Formel 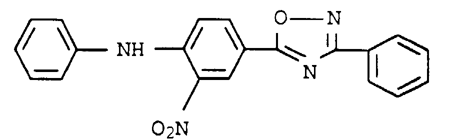 Fp.: 216°C.

### Beispiel 3

a) 183 g 3-Nitro-4-chlorbenzonitril wurden mit 320 ml Isobutanol, 300 ml Wasser, 108 g Soda und 0,5 g eines Schwermetallkomplexbildners versetzt und das Gemisch 5 h bei 80°C gerührt. Danach ließ man absitzen und trennte die wäßrige Phase ab. Anschließend versetzte man die organische Phase mit 90 g Natriumhydrogencarbonat und danach mit 155 g Benzoylchlorid und rührte 30 min bei 60 bis 90°C nach. Danach gab man 250 ml Wasser hinzu, ließ absitzen und trennte dann die Wasserphase erneut ab. Aus der organischen Phase wurde zunächst Isobutanol abdestilliert und der Rückstand auf 170°C erhitzt. Nach Aufarbeiten wie üblich erhielt man 290 g des Produktes der Formel 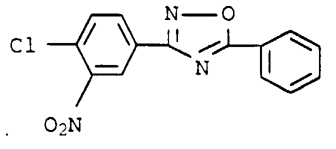 Fp.: 141°C.
b) 302 g des unter a) beschriebenen Produktes wurden mit 180 g m-Xylidin, einer katalytischen Menge Kupferpulver und 60 g N-Methylpyrrolidon versetzt. Dann erhitzte man auf 130°C und gab innerhalb von 1 h 55 g Natriumcarbonat hinzu. Am Schluß erhöhte man die Temperatur auf 165°C. Danach wurde auf 100°C abgekühlt und dann mit verdünnter Salzsäure auf einen pH-Wert von 2 gestellt. Der ausgefallene Farbstoff der Formel 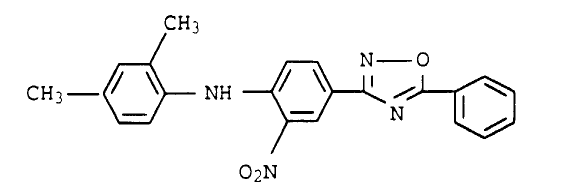 wurde abgesaugt und mit Wasser gewaschen.
   Man erhielt 380 g eines orangefarbenen Pulvers, Fp.: 143°C.
   Der Farbstoff löst sich in Aceton mit gelber Farbe und färbt Polyethylenterephthalatgewebe in kräftig gelbem Ton an. Die Färbungen sind ausgezeichnet (heiß)lichtecht und der Farbstoff zeigt ein gutes färberisches Verhalten.

In analoger Weise können die folgend aufgeführten gelben Farbstoffe erhalten werden.

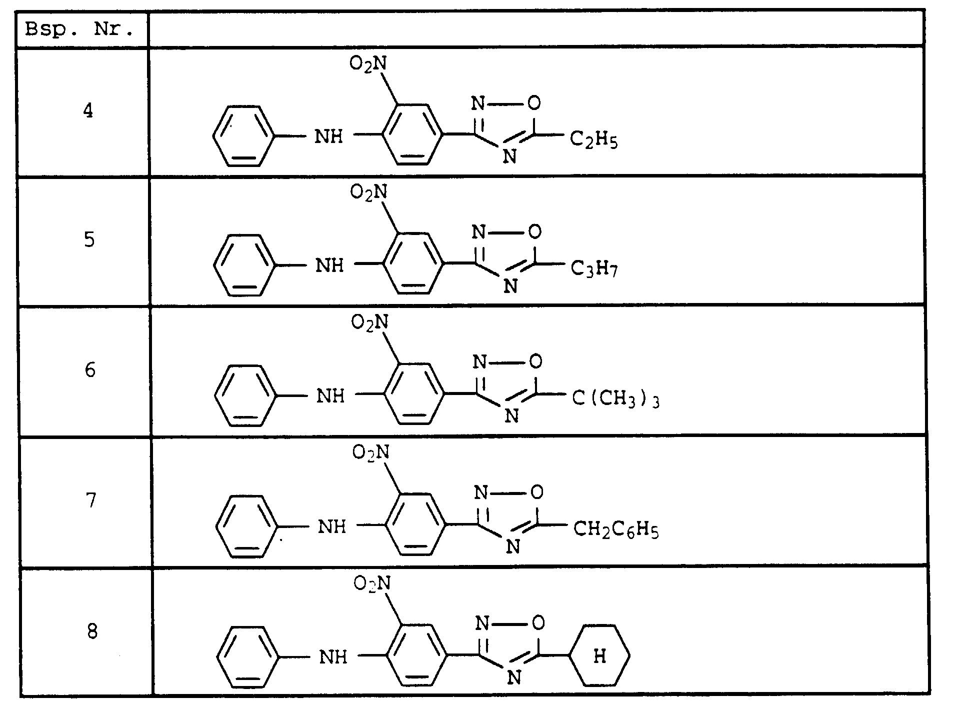

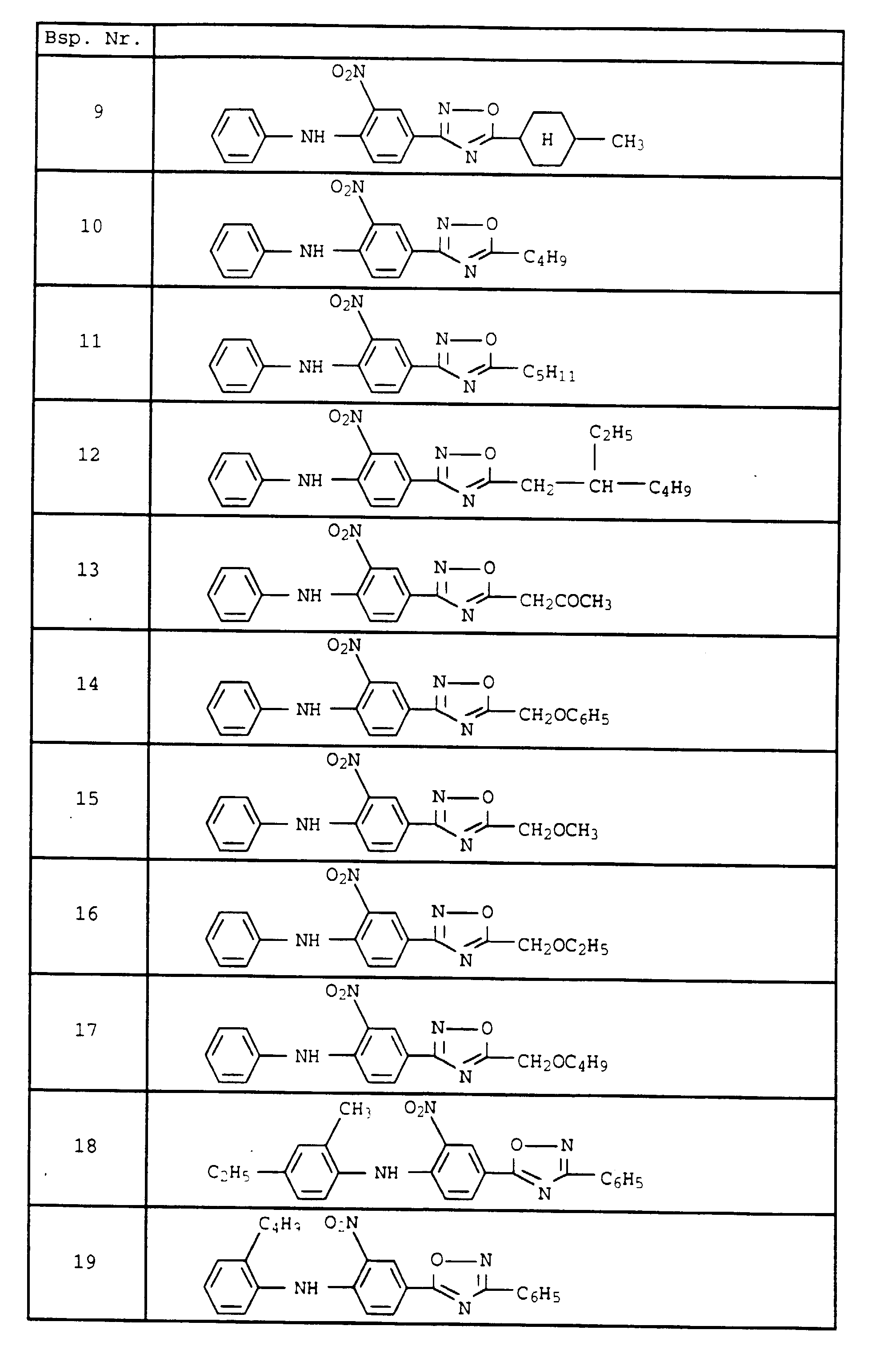

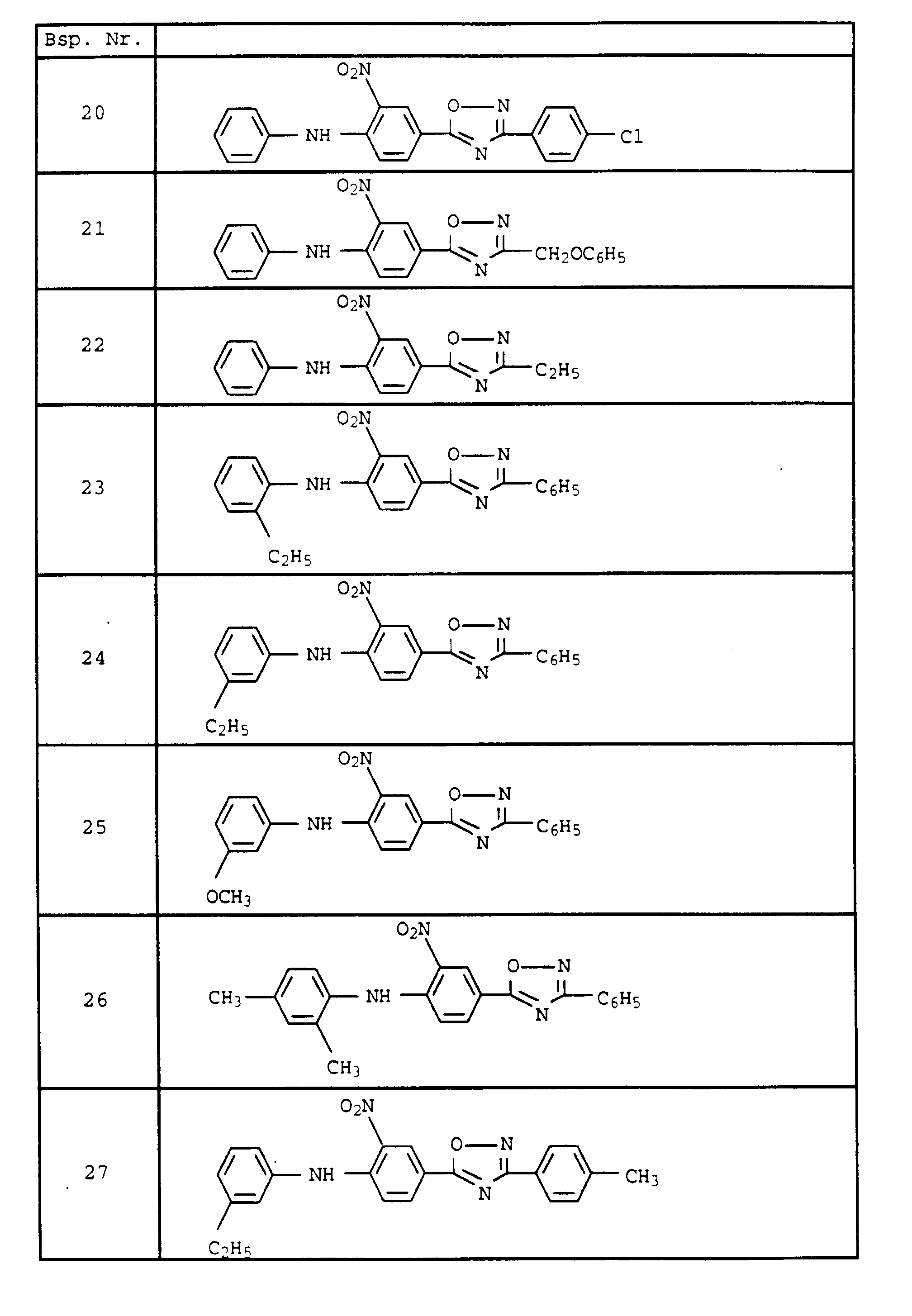

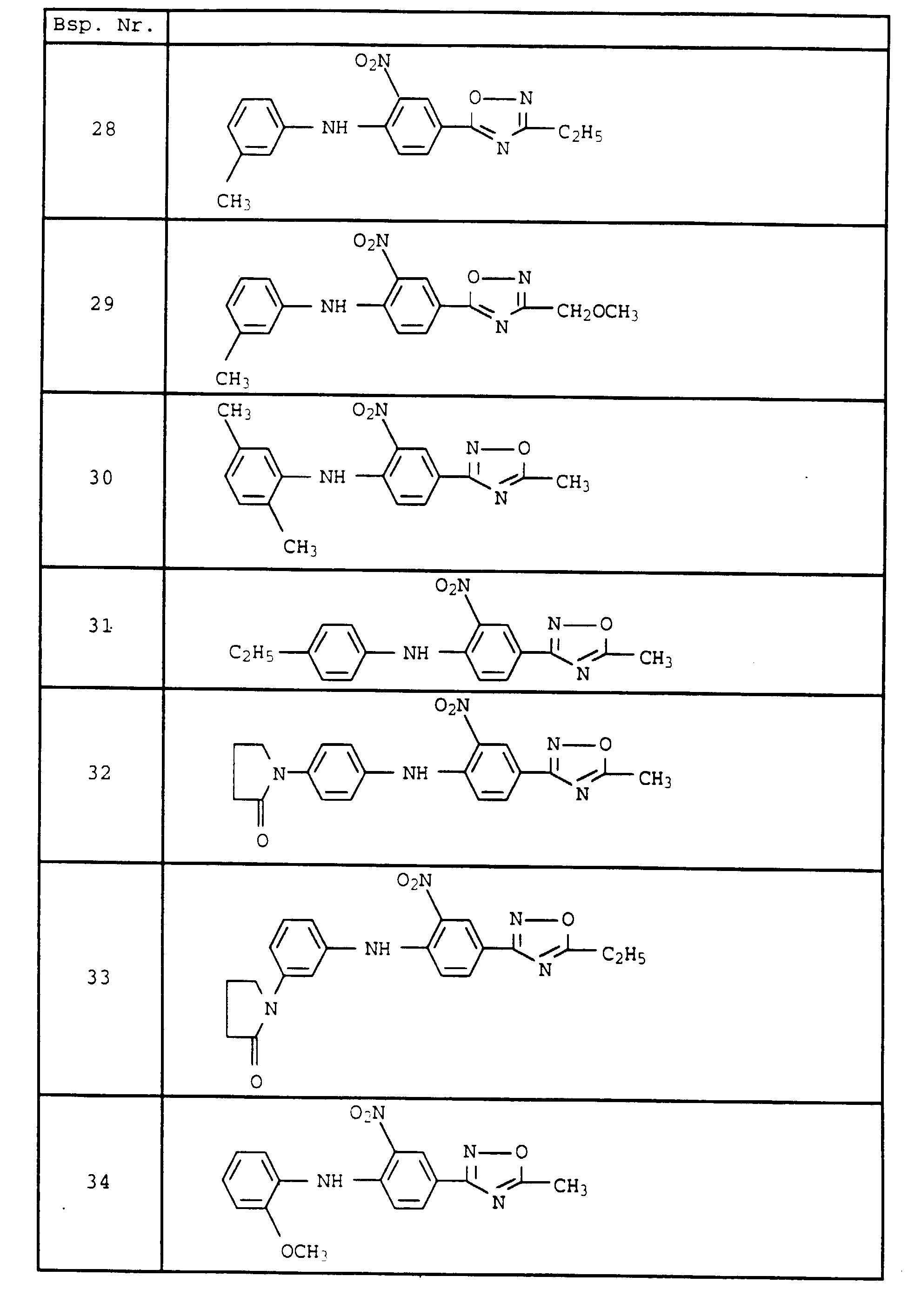

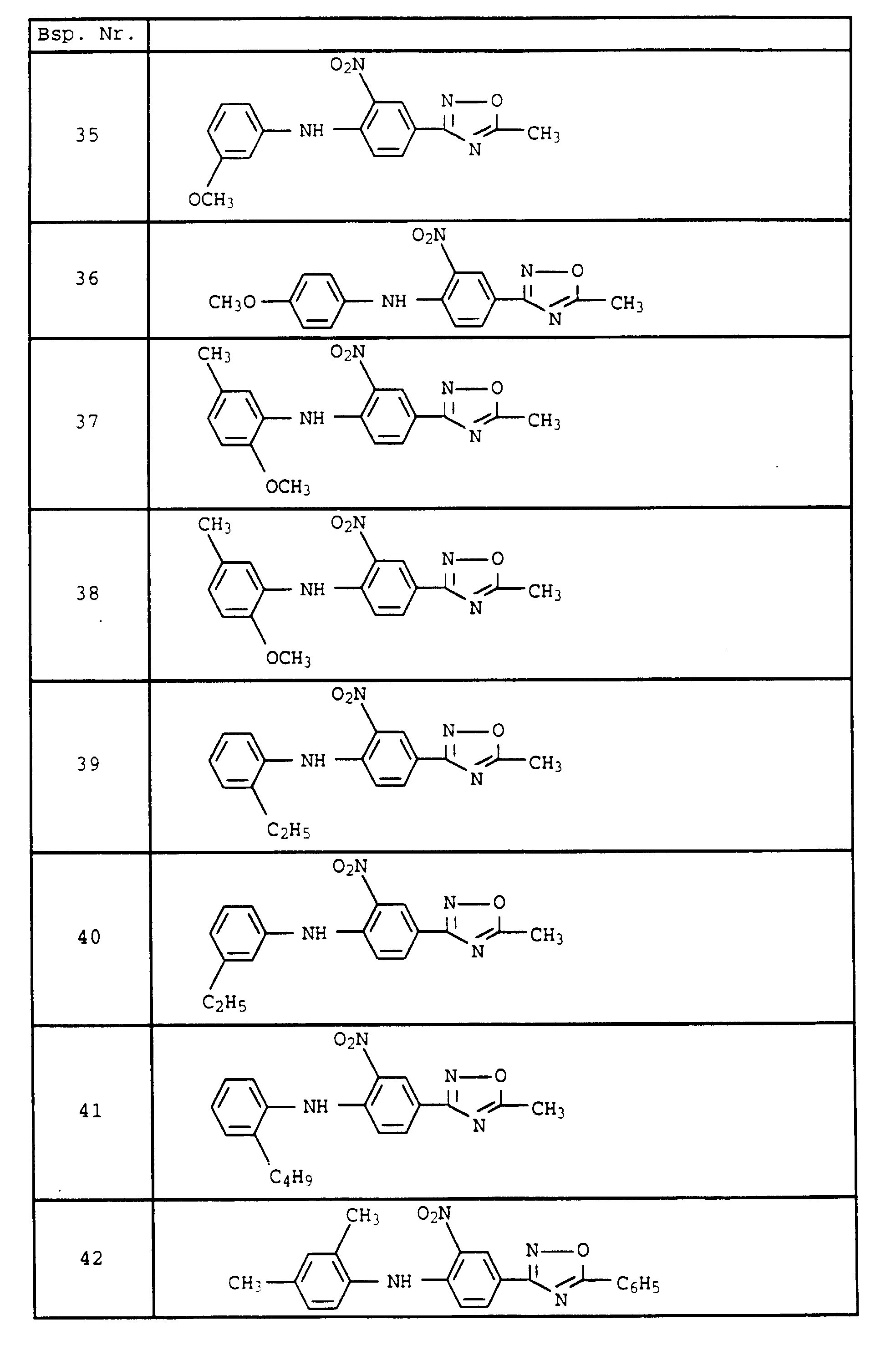

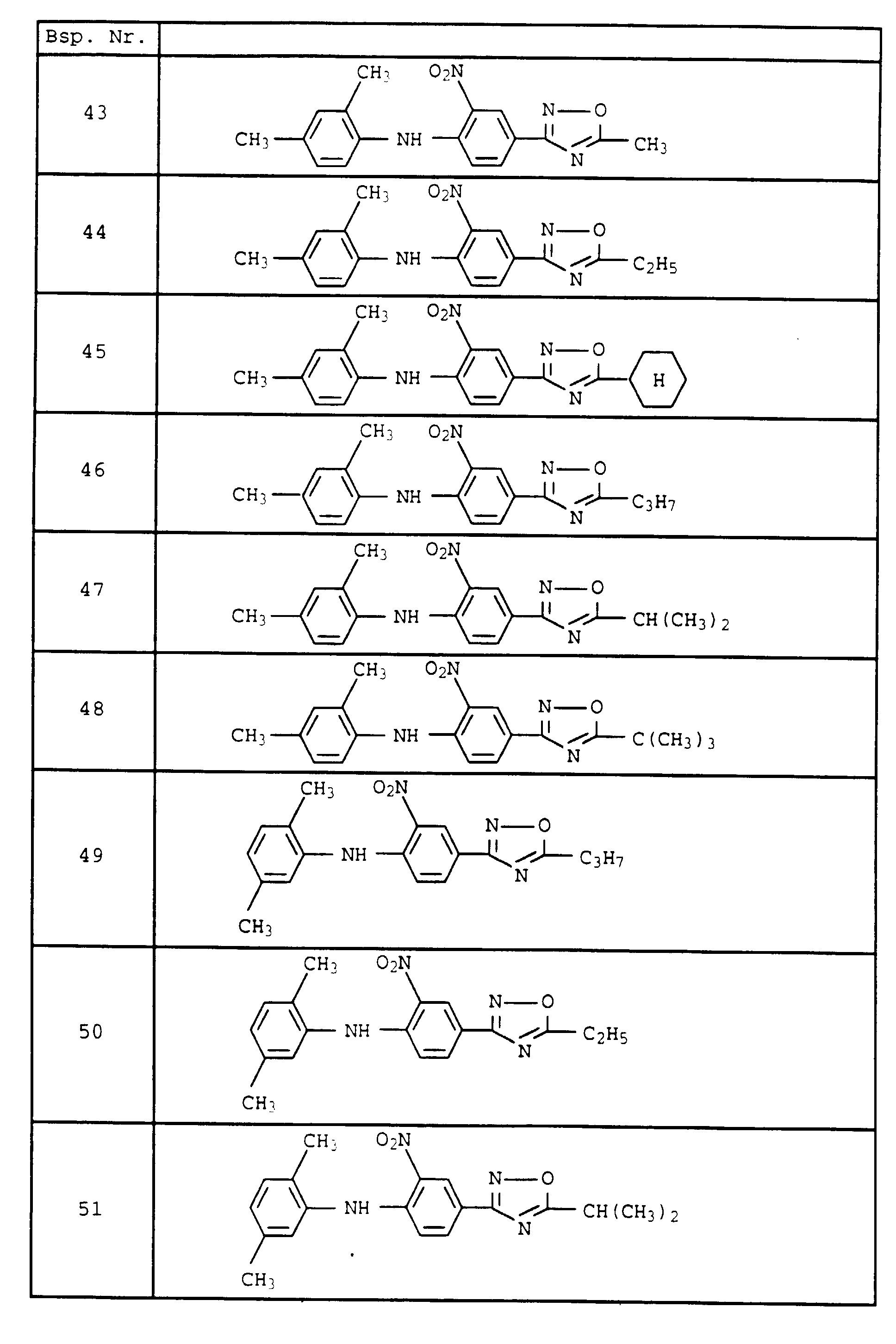

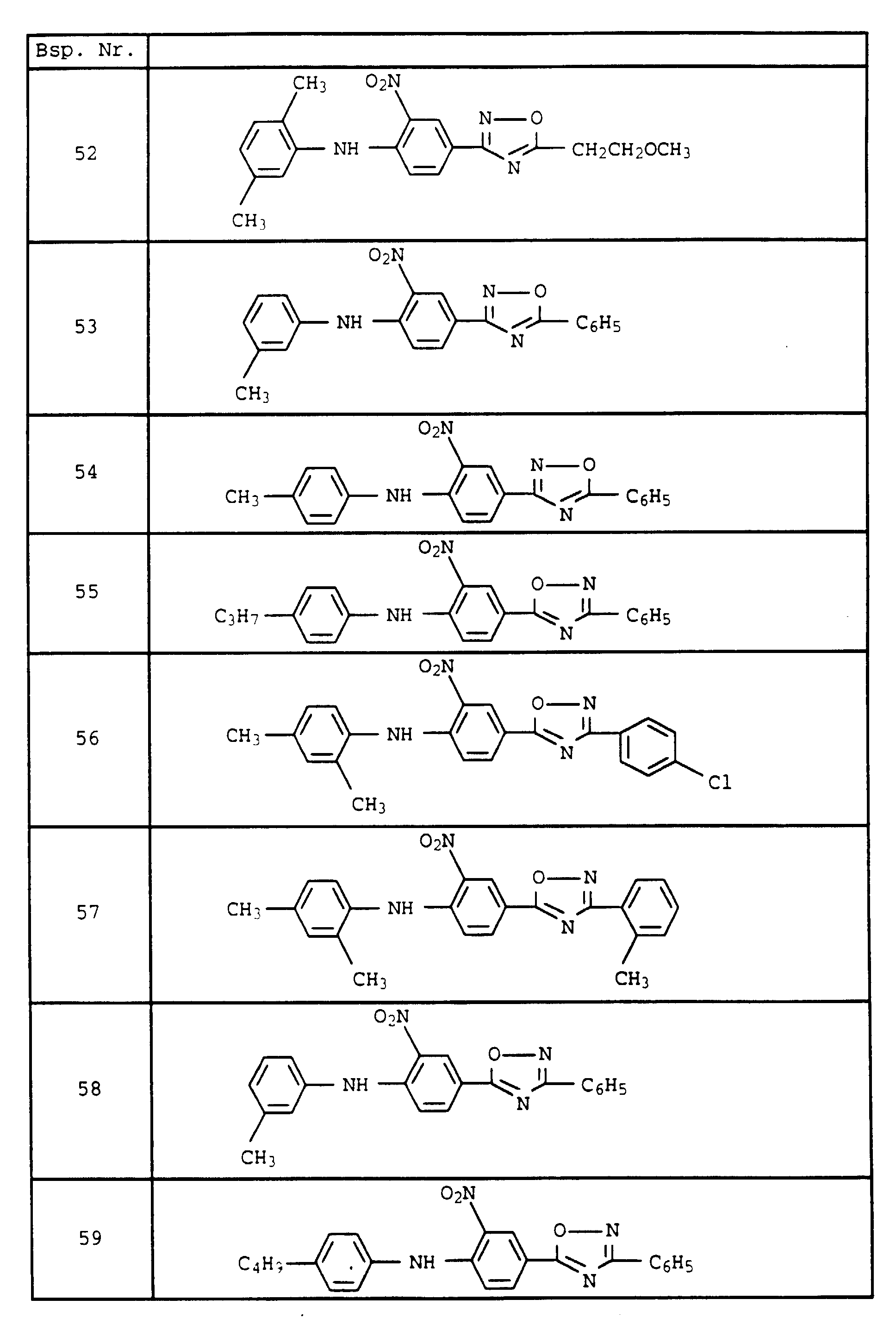

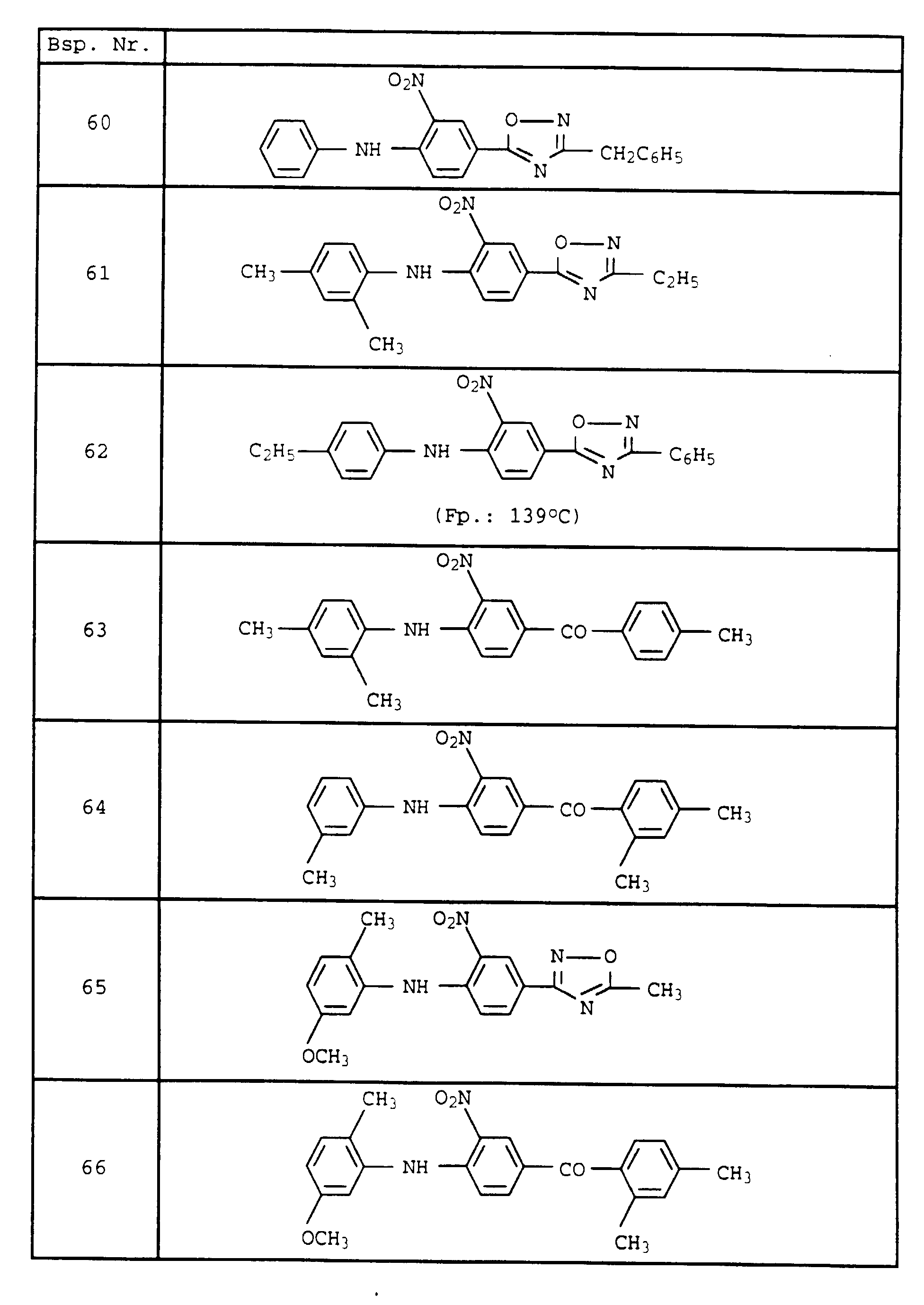

## Patentansprüche

1. Nitrofarbstoffe der Formel I 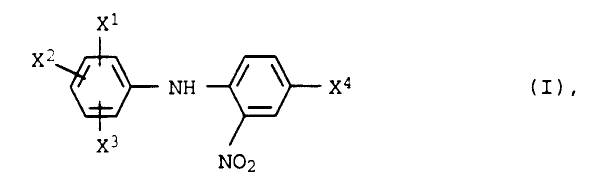 in der
X¹ und X² unabhängig voneinander jeweils Wasserstoff, C₁-C₆-Alkyl, C₁-C₆-Alkoxy oder Pyrrolidin-2-on-1-yl,
X³ Wasserstoff oder C₁-C₆-Alkyl und
X⁴ einen Rest der Formel 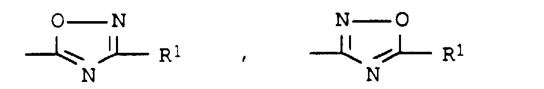 oder 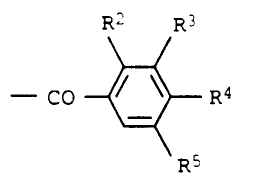 bedeuten, worin
R¹ für Wasserstoff, C₁-C₁₂-Alkyl, das durch 1 bis 3 Sauerstoffatome in Etherfunktion unterbrochen sein kann und gegebenenfalls durch Phenyl, Phenoxy oder Acetyl substituiert ist, C₃-C₇-Cycloalkyl, das gegebenenfalls durch C₁-C₄-Alkyl substituiert ist, oder Phenyl, das gegebenenfalls durch C₁-C₄-Alkyl, C₁-C₄-Alkoxy oder Halogen substituiert ist,
R² und R³ unabhängig voneinander jeweils für Wasserstoff, C₁-C₆-Alkyl oder C₁-C₆-Alkoxy,
R⁴ für Wasserstoff, Methyl, Ethyl, Methoxy oder Ethoxy und
R⁵ für Wasserstoff oder C₁-C₆-Alkyl stehen.

2. Nitrofarbstoffe nach Anspruch 1, dadurch gekennzeichnet, daß X¹ und X² unabhängig voneinander jeweils Wasserstoff oder C₁-C₄-Alkyl und X³ Wasserstoff bedeuten.

3. Nitrofarbstoffe nach Anspruch 1, dadurch gekennzeichnet, daß X⁴ einen Rest der Formel 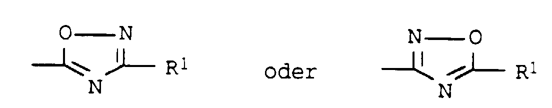 bedeutet, worin R¹ jeweils die in Anspruch 1 genannte Bedeutung besitzt.

4. Nitrofarbstoffe nach Anspruch 1, dadurch gekennzeichnet, daß X⁴ einen Rest der Formel 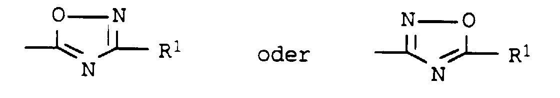 bedeutet, worin R¹ jeweils für C₁-C₄-Alkyl, Cyclohexyl oder Phenyl steht.

5. Nitrofarbstoffe nach Anspruch 1, die der Formel Ia 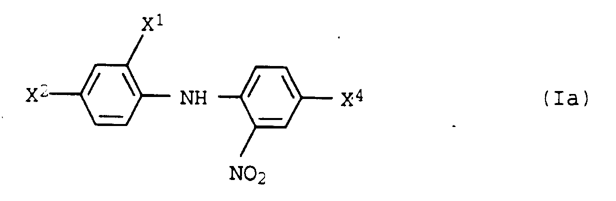 gehorchen, in der X¹, X² und X⁴ jeweils die in Anspruch 1 genannte Bedeutung besitzen.

6. Nitrofarbstoffe nach Anspruch 5, dadurch gekennzeichnet, daß X¹ und X² beide jeweils Wasserstoff oder beide jeweils Methyl oder X¹ Wasserstoff und X² C₁-C₄-Alkyl bedeuten.

7. Verfahren zur Herstellung der Nitrofarbstoffe gemäß Anspruch 1, dadurch gekennzeichnet, daß man ein Nitrobenzol der Formel II 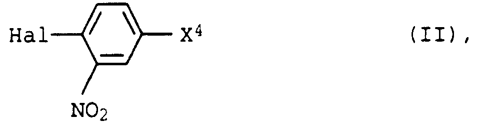 in der Hal Halogen bedeutet und X⁴ die in Anspruch 1 genannte Bedeutung besitzt, mit einem Anilin der Formel III 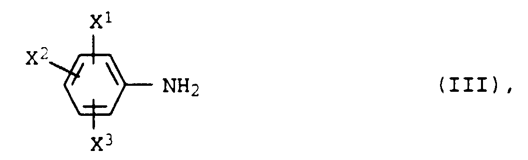 in der X¹, X² und X³ jeweils die in Anspruch 1 genannte Bedeutung besitzen, umsetzt.

8. Verfahren zur Herstellung von Nitrofarbstoffen der Formel I gemäß Anspruch 1, in der X⁴ einen Rest der Formel 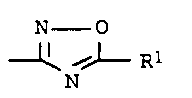 bedeutet, dadurch gekennzeichnet, daß man eine Cyanoverbindung der Formel X 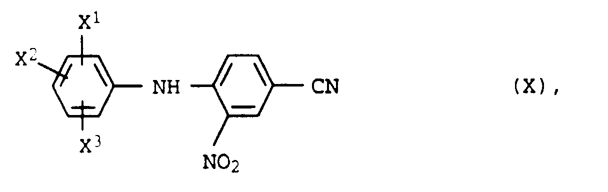 in der X¹, X² und X³ jeweils die in Anspruch 1 genannte Bedeutung besitzen, mit Hydroxylamin in das Amidoxim der Formel XI 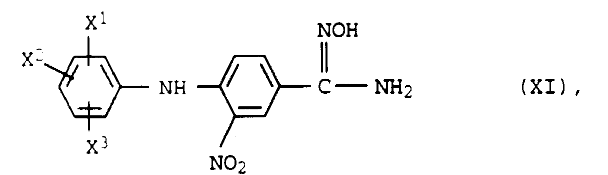 in der X¹, X² und X³ jeweils die in Anspruch 1 genannte Bedeutung besitzen, überführt und anschließend mit einem Carbonsäurederivat der Formel VIII
R¹― CO ― Z (VIII),
in der Z Halogen, C₁-C₆-Alkoxy oder einen Rest der Formel O―CO―R¹ bedeutet und R¹ jeweils die in Anspruch 1 genannte Bedeutung besitzt, zur Reaktion bringt, wobei man die Umsetzung in einem Isobutanol/Wasser-Gemisch ohne Zwischenisolierung des Amidoxims der Formel XI vornimmt.

9. Verwendung der Nitrofarbstoffe gemäß Anspruch 1 zum Färben oder Bedrucken von textilen Materialien.

## Claims

1. Nitro dyes of the formula I 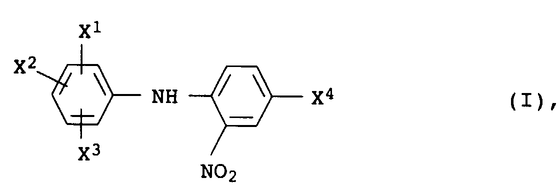 where
X¹ and X² are independently of each other hydrogen, C₁-C₆-alkyl, C₁-C₆-alkoxy or pyrrolidin-2-on-1-yl,
X³ is hydrogen or C₁-C₆-alkyl, and
X⁴ is a radical of the formula 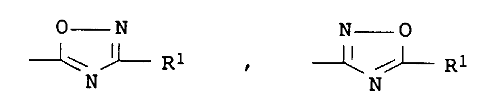 or 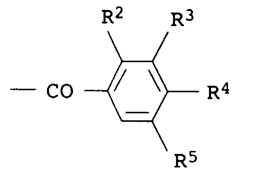 where
R¹ is hydrogen, C₁-C₁₂-alkyl with or without interruption by from 1 to 3 oxygen atoms in ether function and with or without phenyl, phenoxy or acetyl substitution, C₃-C₇-cycloalkyl, which may be C₁-C₄-alkyl-substituted, or phenyl, which may be C₁-C₄-alkyl-, C₁-C₄-alkoxy- or halogen-substituted,
R² and R³ are independently of each other hydrogen, C₁-C₆-alkyl or C₁-C₆-alkoxy,
R⁴ is hydrogen, methyl, ethyl, methoxy or ethoxy, and
R⁵ is hydrogen or C₁-C₆-alkyl.

2. Nitro dyes as claimed in claim 1 wherein X¹ and X² are independently of each other hydrogen or C₁-C₄-alkyl and X³ is hydrogen.

3. Nitro dyes as claimed in claim 1, wherein X⁴ is a radical of the formula 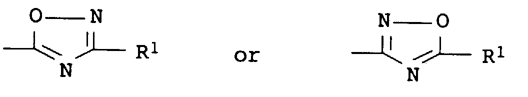 where R¹ is in each case as defined in claim 1.

4. Nitro dyes as claimed in claim 1, wherein X⁴ is a radical of the formula 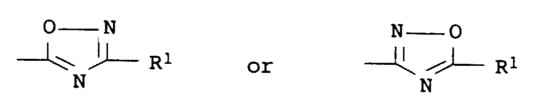 where R¹ is in each case C₁-C₄-alkyl, cyclohexyl or phenyl.

5. Nitro dyes as claimed in claim 1, of the formula Ia 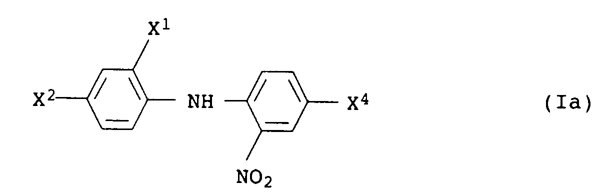 where X¹, X² and X⁴ are each as defined in claim 1.

6. Nitro dyes as claimed in claim 5 where X¹ and X² are both hydrogen or both methyl or X¹ is hydrogen and X² is C₁-C₄-alkyl.

7. A process for preparing the nitro dyes of claim 1, which comprises reacting a nitrobenzene of the formula II 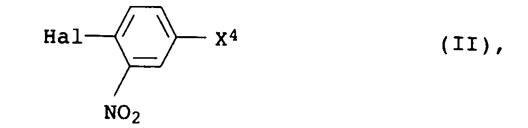 where Hal is halogen and X⁴ is as defined in claim 1, with an aniline of the formula III 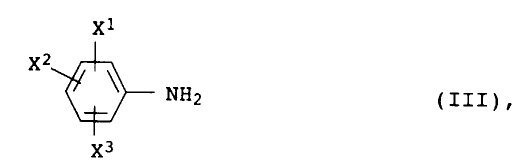 where X¹, X² and X³ are each as defined in claim 1.

8. A process for preparing nitro dyes as claimed in claim 1 of the formula I where X⁴ is a radical of the formula 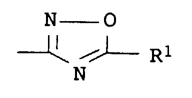 which comprises converting a cyano compound of the formula X 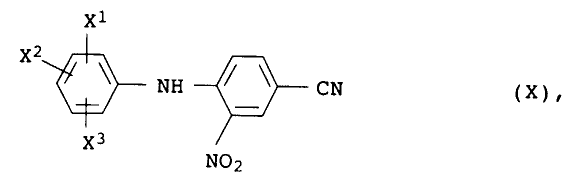 where X¹, X² and X³ are each as defined in claim 1, with hydroxylamine into the amide-oxime of the formula XI 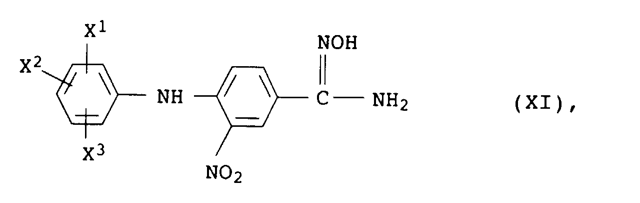 where X¹, X² and X³ are each as defined in claim 1, and then reacting said amide-oxime with a carboxylic acid derivative of the formula VIII
R¹―CO―Z (VIII),
where Z is halogen, C₁-C₆-alkoxy or a radical of the formula O-CO-R¹ and R¹ is in each case as defined in claim 1, the reaction being carried out in an isobutanol-water mixture without intermediately isolating the amide-oxime of the formula XI.

9. The use of the nitro dyes of claim 1 for dyeing or printing textile materials.

## Revendications

1. Colorants nitrés de formule I 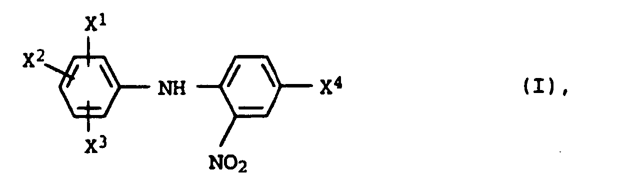 dans laquelle
X¹ et X² représentent chacun indépendamment l'un de l'autre un atome d'hydrogène, un groupement alkyle en C₁-C₆, alcoxy en C₁-C₆ ou pyrrolidin-2-on-1-yle,
X³ représente un atome d'hydrogène ou un groupement alkyle en C₁-C₆ et
X⁴ représente un reste de formule 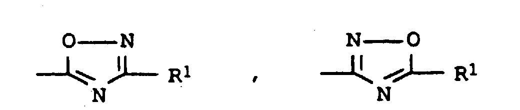 ou 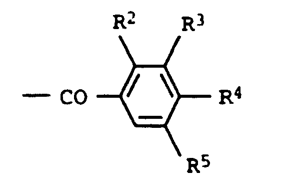 dans lesquelles
R¹ représente un atome d'hydrogène, un groupement alkyle en C₁-C₁₂ pouvant être interrompu par 1 à 3 atomes d'oxygène en fonction éther et étant éventuellement substitué par des groupements phényle, phénoxy ou acétyle, un groupement cycloalkyle en C₃-C₇ éventuellement substitué par des groupements alkyle en C₁-C₄, ou un groupement phényle éventuellement substitué par des groupements alkyle en C₁-C₄, alcoxy en C₁-C₄ ou par des atomes d'halogène,
R² et R³ représentent chacun indépendamment les uns des autres un atome d'hydrogène, un groupement alkyle en C₁-C₆ ou alcoxy en C₁-C₆,
R⁴ représente un atome d'hydrogène, un groupement méthyle, éthyle, méthoxy ou éthoxy et
R⁵ représente un atome d'hydrogène ou un groupement alkyle en C₁-C₆.

2. Colorants nitrés selon la revendication 1, caractérisés en ce que X¹ et X² représentent chacun indépendamment l'un de l'autre, un atome d'hydrogène ou un groupement alkyle en C₁-C₄ et X³ représente un atome d'hydrogène.

3. Colorants nitrés selon la revendication 1, caractérisés en ce que X⁴ représente un reste de formule 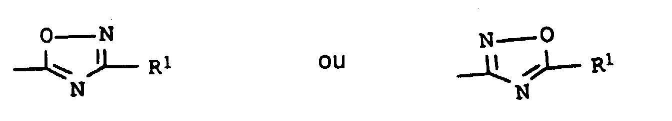 dans lesquelles R¹ prend à chaque fois la signification mentionnée dans la revendication 1.

4. Colorants nitrés selon la revendication 1, caractérisés en ce que X⁴ représente un reste de formule 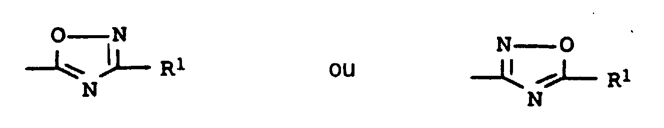 dans lesquelles R¹ représente à chaque fois un groupement alkyle en C₁-C₄, cyclohexyle ou phényle.

5. Colorants nitrés selon la revendication 1 correspondant à la formule la 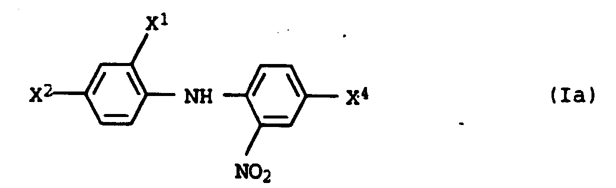 dans laquelle X¹, X² et X⁴ prennent chacun la signification mentionnée dans la revendication 1.

6. Colorants nitrés selon la revendication 5, caractérisés en ce que X¹ et X² représentent tous les deux à chaque fois un atome d'hydrogène ou, tous les deux à chaque fois un groupement méthyle, ou bien X¹ représente un atome d'hydrogène et X² un groupement alkyle en C₁-C₄.

7. Procédé de préparation de colorants nitrés selon la revendication 1, caractérisé en ce que l'on fait réagir un nitrobenzène de formule II 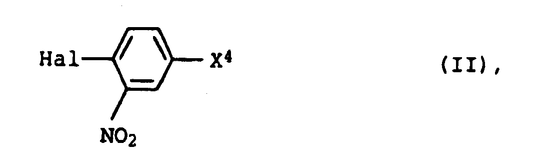 dans laquelle Hal représente un atome d'halogène et X⁴ prend la signification mentionnée dans la revendication 1, avec une aniline de formule III 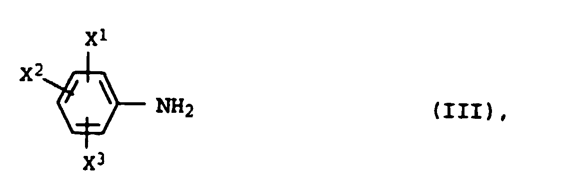 dans laquelle X¹, X² et X³ prennent chacun la signification mentionnée dans la revendication 1.

8. Procédé de préparation de colorants nitrés de formule I selon la revendication 1, dans laquelle X⁴ représente un reste de formule 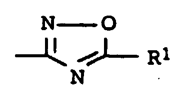 caractérisé en ce que l'on convertit un composé cyano de formule X 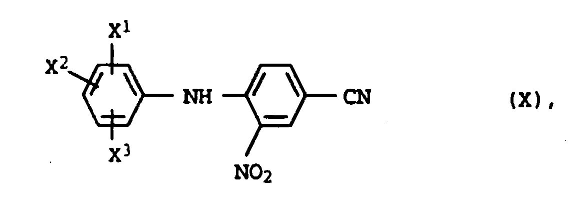 dans laquelle X¹, X² et X³ prennent chacun la signification mentionnée dans la revendication 1, à l'aide d'hydroxylamine, en l'amidoxime de formule XI 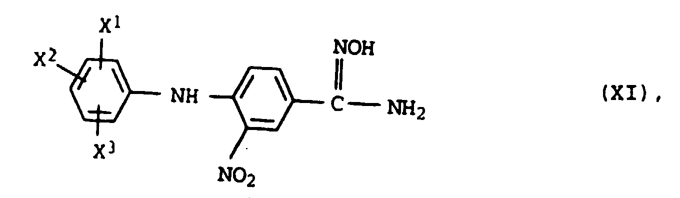 dans laquelle X¹, X² et X³ prennent chacun la signification mentionnée dans la revendication 1, et on la fait ensuite réagir avec un dérivé d'acide carboxylique de formule VIII
R¹―CO―Z (VIII),
dans laquelle Z représente un atome d'halogène, un groupement alcoxy en C₁-C₆ ou un reste de formule O-CO-R¹ et R¹ prend à chaque fois la signification mentionnée dans la revendication 1, la réaction étant effectuée dans un mélange isobutanol/eau sans isolement intermédiaire de l'amidoxime de formule XI.

9. Utilisation de colorants nitrés selon la revendication 1 pour la coloration ou l'impression de matériaux textiles.
